(19) Europäisches Patentamt
European Patent Office
Office européen des brevets

(11) **EP 4 388 985 A1**

(12) **EUROPEAN PATENT APPLICATION**

(43) Date of publication:
**26.06.2024 Bulletin 2024/26**

(21) Application number: **23153931.3**

(22) Date of filing: **30.01.2023**

(51) International Patent Classification (IPC):
***A61B 5/00*** *(2006.01)*    ***A61B 5/024*** *(2006.01)*

(52) Cooperative Patent Classification (CPC):
**A61B 5/02405; A61B 5/4812; A61B 5/4815; A61B 5/4818; A61B 5/7221; A61B 5/7267**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 US 202263434973 P**

(71) Applicant: **Koninklijke Philips N.V.**
**5656 AG Eindhoven (NL)**

(72) Inventors:
• **VAN GORP, Hans**
**5656AG Eindhoven (NL)**
• **FERREIRA DOS SANTOS DA FONSECA, Pedro Miguel**
**5656AG Eindhoven (NL)**
• **VAN GILST, Merel Marietje**
**5656AG Eindhoven (NL)**
• **OVEREEM, Sebastiaan**
**5656AG Eindhoven (NL)**
• **VAN SLOUN, Ruud Johannes Gerardus**
**5656AG Eindhoven (NL)**

(74) Representative: **Philips Intellectual Property & Standards**
**High Tech Campus 52**
**5656 AG Eindhoven (NL)**

(54) **SYSTEM FOR ESTIMATING UNCERTAINTY OF OVERNIGHT SLEEP PARAMETERS THROUGH A STOCHASTIC NEURAL NETWORK**

(57) A sleep monitoring system (100, 500), including: a sensor (114) to sense physiological changes of a subject during a sleep study period and form corresponding sleep study information; a stochastic neural network (129, 329) to: receive the sleep study information; and derive a plurality of sleep sample evaluations each different from each other and based on a same sampling of the received sleep study information; a controller (120, 190, 194) to: receive the plurality of sleep sample evaluations and calculate a robust estimate of one or more sleep parameters thereon; calculate an estimate of uncertainty for at least one of the one or more sleep parameters; determine whether the estimate of uncertainty for the at least one of the one or more sleep parameters is greater than a threshold value; and render the estimate of uncertainty on a rendering device (192) of the system based upon the determination.

FIG. 5

EP 4 388 985 A1

**Description**

FIELD OF THE PRESENT SYSTEM

The present system relates to an automatic sleep scoring system that estimates uncertainty in overnight sleep statistics and, more particularly, an automatic sleep scoring system that estimates uncertainty in overnight sleep parameters using a stochastic neural network, and a method of operation thereof.

BACKGROUND OF THE PRESENT SYSTEM

Sleep stage scoring is the process whereby a sleep measurement acquired, typically with polysomnography (PSG), is analyzed and split into 30-second segments called epochs. Each epoch is assigned a sleep stage, resulting in a hypnogram. The hypnogram serves as a crucial diagnostic tool for several sleep disorders. The hypnogram is crucial not only on its own, but also because several overnight sleep parameters may be calculated from it, such as sleep onset latency (SOL), and total sleep time (TST). In the same manner, PSG or other sleep recordings may be analyzed for the scoring of events, such as sleep-disordered breathing (SDB) which includes obstructive-sleep apnea (OSA), central-sleep apneas (CSA), and hypopneas. These scored events may be used, together with statistics about TST, to indicate the severity of sleep apnea in composite scores such as the apnea-hypopnea index (AHI).

The gold standard for sleep scoring is for experienced human scorers to perform sleep staging and annotate sleep events on polysomnography (PSG) data. However, this process is laborintensive, leading to the development of partially-automated alternatives. Moreover, sleep staging and event scoring may also be performed on alternative measurements, e.g., photoplethysmography (PPG), actigraphy, and suprasternal pressure. The advantage of these methods is that they allow for longitudinal and ambulatory sleep studies. However, human scorers cannot evaluate these surrogate measurements.

One well-known challenge in sleep staging is the low inter-rater agreement for human scorers, leading to uncertainty about the hypnogram. For example, multiple sleep experts will score a given hypnogram slightly differently leading to uncertainty about overnight sleep parameters such as total sleep time and the apnea-hypopnea index. Inter-rater agreement/reliability is a measure of consistency used to evaluate the extent to which different judges agree in their assessment decisions. Inter-rater reliability is essential when making decisions in clinical settings. If inter-rater reliability is weak, it can have detrimental effects and lead to uncertainty in a diagnosis and treatment plan.

Due to this variability in assessing sleep, there is also uncertainty about overnight sleep parameters. Existing automated sleep staging algorithms, such as hypnodensity, can capture short-term uncertainty on a per-epoch basis. However, these algorithms lack the long-term modeling required to also permit uncertainty analysis of sleep parameters over the whole night. The same is true for the scoring of SDB events and the estimation of AHI, with human scorers often disagreeing on the number of apnea and hypopnea events on the same recording, leading to agreement (e.g., measured with intraclass correlation coefficient) as low as 84%.

It is difficult to assess the quality and/or uncertainty of a hypnogram created by an automated model using conventional methods. Further, conventional automatic sleep scoring algorithms do not allow for the estimation of uncertainty over overnight sleep parameters.

Accordingly, embodiments of the present system provide features and advantages which obviate conventional sleep study and scoring systems and methods. To overcome the aforementioned barriers and detriments as well as others, there is a need for a system which can reliably and inexpensively provide an accurate sleep scoring system thus overcoming the aforementioned barriers and detriments as well as others of conventional systems.

SUMMARY OF THE PRESENT SYSTEM

The system(s), device(s), method(s), arrangements(s), interface(s), computer program(s), processes, etc., (hereinafter each of which will be referred to as system, unless the context indicates otherwise), described herein address problems in prior art systems.

In accordance with embodiments of the present system, there is disclosed a sleep monitoring system, including: at least one sensor configured to sense physiological changes of a subject during a sleep study period having a plurality of epochs and form corresponding sleep study information for the plurality of epochs during the sleep study; a stochastic neural network configured to: receive the sleep study information for the plurality of the epochs during the sleep study period from the at least one sensor; and derive a plurality of sleep sample evaluations each different from each other and based on a same sampling of the received sleep study information; at least one controller (120, 190, 194) configured to: receive the plurality of sleep sample evaluations and calculate a robust estimate of one or more sleep parameters thereon; calculate an estimate of uncertainty for at least one of the one or more sleep parameters; determine whether the estimate of uncertainty for the at least one of the one or more sleep parameters is greater than a threshold value;

and render the estimate of uncertainty on a rendering device (192) of the system based upon the determination. The plurality of sleep sample evaluations may be a plurality of hypnograms, wherein the at least one controller may be further configured to analyze the plurality of hypnograms to determine a total number of obstructive and central apneas and hypopneas, and total sleep time (TST) for each of the plurality of hypnograms. The at least one controller may be further configured to receive the plurality of sleep sample evaluations and calculate a robust estimate of two or more sleep parameters thereon and calculate an estimate of uncertainty for at least two of the two or more sleep parameters. The at least one controller may be further configured to receive the plurality of sleep sample evaluations covering at least two sleep study nights and calculate a robust estimate of one or more sleep parameters thereon, wherein the at least one controller is further configured to identify a trend of the one or more sleep parameters over time.

The at least one controller may be further configured to repeat the sleep study when the estimate of uncertainty associated with one or more of the one or more sleep parameters is greater than a threshold value. The plurality of sleep sample evaluations may be at least one of: A- (temporal) location (i.e., when, during night they occur) and duration of arousals; B - location and duration of SDB events (including a possible differentiation between these, e.g., between obstructive apneas, hypopneas, central apneas); C- location, duration and degree of oxygen desaturations; D - location, and duration of periodic limb movements; E - a plurality of hypnograms, wherein the at least one controller may be further configured to calculate the robust estimate of one or more sleep parameters and to calculate the estimate of uncertainty for each of the plurality of sleep parameters. The at least one controller may be further configured to calculate one or more combinations of the sleep sample evaluations and to calculate a robust estimate of one or more combinations of the sleep sample evaluations to calculate an estimate of uncertainty for each of the plurality of combinations of the sleep sample evaluations. The at least one controller may be further configured to determine whether the aggregated estimate of uncertainty for each of the one or more sleep parameters is greater than a corresponding threshold value. The at least one controller may be further configured to render the estimate of uncertainty on the rendering device of the system based upon the determination of whether the aggregated estimate of uncertainty for one or more of the one or more sleep parameters is greater than the corresponding threshold value. The at least one controller may be further configured to repeat the sleep study when the aggregated estimate of uncertainty associated with the one or more of the one or more sleep parameters is greater than the corresponding threshold value. The at least one controller may be further configured to render the estimate of uncertainty on the rendering device of the system and a recommendation to add at least one additional sensor or substitute a second sensor for one of the at last one sensor based upon a determination that the aggregated estimate of uncertainty for one or more of the one or more sleep parameters is greater than a corresponding threshold value, wherein the at least one controller is further configured to repeat the sleep study with the added at least one additional sensor or the substituted second sensor when the aggregated estimate of uncertainty associated with the one or more of the one or more sleep parameters is greater than the corresponding threshold value.

In accordance with embodiments of the present system, there is disclosed a sleep monitoring system, including: at least one sensor configured to sense physiological changes of a subject during a sleep study period having a plurality of epochs and form corresponding sleep study information for the plurality of epochs during the sleep study, wherein the sleep study information comprises a plurality of channels; a stochastic neural network configured to: receive the sleep study information for the plurality of the epochs during the sleep study period from the at least one sensor; and derive a plurality of hypnograms each different from each other and conditioned upon the received sleep study information; at least one controller configured to: receive the plurality of sleep sample evaluations and calculate a robust estimate of one or more sleep parameters thereon; calculate an estimate of uncertainty for at least one of the one or more sleep parameters; determine whether the estimate of uncertainty for the at least one of the one or more sleep parameters is greater than a threshold value; render the determination when the estimate of uncertainty for the at least one of the one or more sleep parameters is equal to or less than the threshold value; and delete one of the plurality of channels from the sleep study information for one or more of the plurality of the epochs to derive modified sleep study information when the estimate of uncertainty for one or more of the one or more sleep parameters is determined to be greater than the threshold value, wherein the stochastic neural network is further configured to derive a second plurality of sleep sample evaluations each different from each other and conditioned upon received modified sleep study information, and

wherein the at least one controller is further configured to: receive the second plurality of sleep sample evaluations and calculate a robust modified estimate of one or more sleep parameters thereon, calculate a modified estimate of uncertainty for at least one of the one or more modified sleep parameters, determine whether the modified estimate of uncertainty for the at least one of the one or more modified sleep parameters is greater than the threshold value, and render the determination when the modified estimate of uncertainty for the at least one of the one or more modified sleep parameters is equal to or less than the threshold value.

When the modified estimate of uncertainty for the at least one of the one or more modified sleep parameters is greater than the threshold value, the at least one controller may be further configured to replace the one of the plurality of channels back into the sleep study information and delete a second one of the plurality of channels from the sleep study information, the stochastic neural network is further configured to derive a third plurality of sleep sample evaluations each different from each other and conditioned upon received further modified sleep study information, and the at least one controller may be further configured to determine whether a further modified estimate of uncertainty for the at least one of the one or more further modified sleep parameters is greater than the threshold value. The at least one controller and the stochastic neural network may be further configured to replace and utilize different ones of the plurality of channels until each one of the plurality of channels have been replaced unless a resultant estimate of uncertainty for the at least one of the one or more sleep parameters is equal to or less than the threshold value. The at least one controller may be further configured to receive the plurality of sleep sample evaluations and calculate a robust estimate of two or more sleep parameters thereon and calculate an estimate of uncertainty for at least two of the two or more sleep parameters.

In accordance with embodiments, the at least one controller may be further configured to receive the plurality of sleep sample evaluations covering at least two sleep study nights and calculate a robust estimate of one or more sleep parameters thereon, wherein the at least one controller is further configured to identify a trend of the one or more sleep parameters over time. The plurality of sleep sample evaluations may be at least one of: A- (temporal) location (i.e., when, during night they occur) and duration of arousals; B - location and duration of SDB events (including a possible differentiation between these, e.g., between obstructive apneas, hypopneas, central apneas); C- location, duration and degree of oxygen desaturations; D - location, and duration of periodic limb movements; E - a plurality of hypnograms, wherein the at least one controller is further configured to calculate the robust estimate of one or more sleep parameters and to calculate the estimate of uncertainty for each of the plurality of sleep parameters. The at least one controller may be further configured to calculate one or more combinations of the sleep sample evaluations and to calculate a robust estimate of one or more combinations of the sleep sample evaluations to calculate an estimate of uncertainty for each of the plurality of combinations of the sleep sample evaluations. The at least one controller may be further configured to determine whether the aggregated estimate of uncertainty for each of the one or more sleep parameters is greater than a corresponding threshold value. The at least one controller may be further configured to render the estimate of uncertainty on the rendering device of the system based upon the determination of whether the aggregated estimate of uncertainty for one or more of the one or more sleep parameters is greater than the corresponding threshold value.

BRIEF DESCRIPTION OF THE DRAWINGS

It should be expressly understood that the drawings are included for illustrative purposes and do not represent the scope of the present system. It is to be understood that the figures may not be drawn to scale. Further, the relation between objects in a figure may not be to scale and may in fact have a reverse relationship as to size. The figures are intended to bring understanding and clarity to the structure of each object shown, and thus, some features may be exaggerated in order to illustrate a specific feature of a structure. In the accompanying drawings, like reference numbers in different drawings may designate identical or similar elements, portions of similar elements and/or elements with similar functionality. The present system is explained in further detail, and by way of example, with reference to the accompanying drawings which show features of various exemplary embodiments that may be combinable and/or severable wherein:

> FIG. 1 illustratively shows a schematic block diagram of a portion of a system operating in accordance with embodiments of the present system;
> FIG. 2 illustratively shows a functional flow diagram performed by a process in accordance with embodiments of the present system;
> FIG. 3 illustratively shows a schematic block diagram of a portion of a system operating in accordance with embodiments of the present system;
> FIG. 4 illustratively shows a schematic block diagram of a portion of a system operating in accordance with embodiments of the present system; and
> FIG. 5 illustratively shows an automated sleep study system for generating uncertainty estimates in accordance with embodiments of the present system.

DETAILED DESCRIPTION OF THE PRESENT SYSTEM

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well-known devices, circuits, tools, techniques and methods are omitted so as not to obscure the description of the present system.

The term "and/or," and formatives thereof, should be understood to mean that only one or more of the recited elements may need to be suitably present (e.g., only one recited element is present, two of the recited elements may be present, etc., up to all of the recited elements may be present) in a system in accordance with the claims recitation and in accordance with one or more embodiments of the present system. In the context of the present embodiments, the terms "about", substantially and "approximately" denote an interval of accuracy that a person skilled in the art will understand to still ensure the technical effect of the feature in question which in some cases may also denote "within engineering tolerances." For example, the terms may indicate a "deviation" from indicated numerical value(s) of $\pm 20$ %, $\pm 15$ %, $\pm 10$ %, $\pm 5$ %, $\pm 1$ % $\pm 0.5$ % or $\pm 0.1$ %.

The terms clinician, clinicians, or formatives thereof, may include a professional such as a doctor, a clinician, a technologist, an operator, an expert, a nurse, a medical specialist, a technician, a prescriber, and/or the like, who may operate and/or review information related to the patient such as system setting information (SSI), system information, and/or data generated by embodiments of the present system and associated data for review by a clinician, an expert, a medical specialist, a doctor, an operator, a technician, and/or the like unless the context indicates otherwise. The terms patient, patients, or formatives thereof, may include patients or other individuals (e.g., persons, subjects, subjects under test, etc.) or other users who may be receiving any type of sleep apnea monitoring or assessment using systems, machines, processes, and/or methods operating in accordance with embodiments of the present system.

The Apnea-Hypopnea Index (AHI) is an index used to indicate the severity of sleep apnea. It is represented by the number of apnea and hypopnea events per hour of sleep. In accordance with embodiments of the present system, an apnea may last for at least ten seconds and be associated with a decrease in blood oxygenation. The AHI values for adults are categorized as follows: (1) Normal: AHI < 5; (2) Mild sleep apnea: $5 \leq$ AHI < 15; (3) Moderate sleep apnea: $15 \leq$ AHI < 30; and (4) Severe sleep apnea: AHI $\geq$ 30. These terms may be referred to as AHI values in the current description.

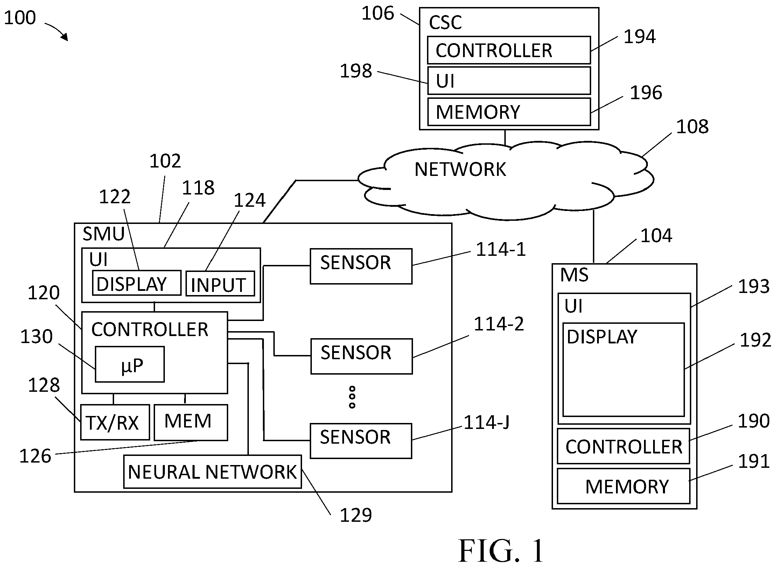

FIG. 1 illustratively shows a schematic block diagram of a portion of a system 100 (hereinafter the system 100) operating in accordance with embodiments of the present system. The system 100 may include one or more of a sleep monitoring unit (SMU) 102, a mobile station (MS) 104, and a clinical service center (CSC) 106, one or more of which may communicate with the other via a network 108 and any suitable communication method or methods such as wired and/or wireless (e.g., optical) communication methods over the network 108.

The network 108 may include any suitable network such as an ad-hoc network, a body area network (BAN), a personal area network (e.g., e.g., Bluetooth™, etc.), a Zigbee network, Wi-Fi, a local area network (LAN), a wide area network (WAN), a telephony network, a cellular network (e.g., 5G, etc.), etc. The SMU 102, the MS 104, the CSC 106 and the network 108 may be controlled by at least one controller (e.g., one or more of controllers 120, 190, 194 and/or one or more others) which may control the overall operation of the system and may include one or more logic devices such as a microprocessor and/or the like.

The at least one controller may access the memory and may obtain system setting information (SSI) which may include one or more of operating parameters (e.g., scan type, etc.), threshold values, warnings, display settings, user information, patient information such as patient identification (ID), content (e.g., video, audio, etc.), etc. The SSI may be set by the system and/or user and may be updated during use. The at least one controller may generate and render an interface with which the patient and/or clinician may, for example, interact with to, for example, set, reset, select, and/or adjust one or more settings of the SSI and/or enter information (e.g., ID, test type, settings, parameters (e.g., test duration), results, etc.) which may be stored in the SSI in a memory of the system.

The MS 104 may include any suitable mobile station(s) such as a smart-phone (e.g., an I-Phone, a Galaxy phone, etc.), a personal digital assistant (PDA), a smart watch (e.g., an Apple™ Watch™, a Galaxy™ watch, etc.), a smart ring (e.g., generally a token) and/or the like, and may include a memory 191, at least one controller 190, a user interface (UI) 193, and a transceiver (Tx/Rx) configured to communicate with the network 108.

The UI 193 may provide an interface with which a user (e.g., the patient) may interact, such interfaces may include, inter alia, a touch-screen display 192, an optical sensor such as a camera, a proximity sensor, a speaker, and/or a microphone. However, other user interfaces are also envisioned. The UI 193 may render information, such as content, etc., on a display and/or a speaker for the convenience of the patient, and may receive information entered by the patient such as selections, requests, commands, patient settings, etc., using any suitable input method such as gesture inputs, a touch input, voice input, etc. The memory 191 may store information for use by the system such as the SSI, sleep monitoring information (SMI), system settings, system parameters, operating parameters, and/or combinations thereof, as discussed herein. The patient identification (ID) may be employed to identify the patient and/or the corresponding test results.

The MS 104 may transmit information such as operating parameters, instructions, requests, commands (e.g., a synchronization command, etc.), to the SMU 102, and may receive information generated by the SMU 102 such as the SSI, operating parameters, and/or combinations thereof, for further processing. The MS 104 may further receive and/or transmit SMI as discussed herein. Data transfer may occur on a periodic or non-periodic intervals as may be set forth in the SSI or requested by the system (e.g., the SMU 102, the CSC 106, and/or the MS 104), patient, and/or clinician. For the sake of clarity, only a single MS 104 is discussed unless the context indicates otherwise. However, without limitation other MSs may be employed by the system. For example, a clinician such as a doctor may have an MS which may receive information such as SSI, SMI, etc., from other portions of the system for further review, processing, and/or storage.

The CSC 106 may include one or more of a controller 194, a memory 196, and a UI 198. The controller 194 may control the overall operation of the CSC 106 and, as such may receive information, such as the SSI, SMI, and/or other information from the SMU 102 and/or MS 104 for further processing. The CSC 106 may further communicate with the SMU 102 and/or MS 104 to, for example, transmit diagnostic results of the processing (e.g., apnea detected, apnea not detected, etc.) to be rendered on a rendering device of the MS 104 for the convenience of the user. The diagnostic results may be stored in the SMI in a memory of the system. The CSC 106 may process data in real time or may obtain data that has been stored in a memory of the system.

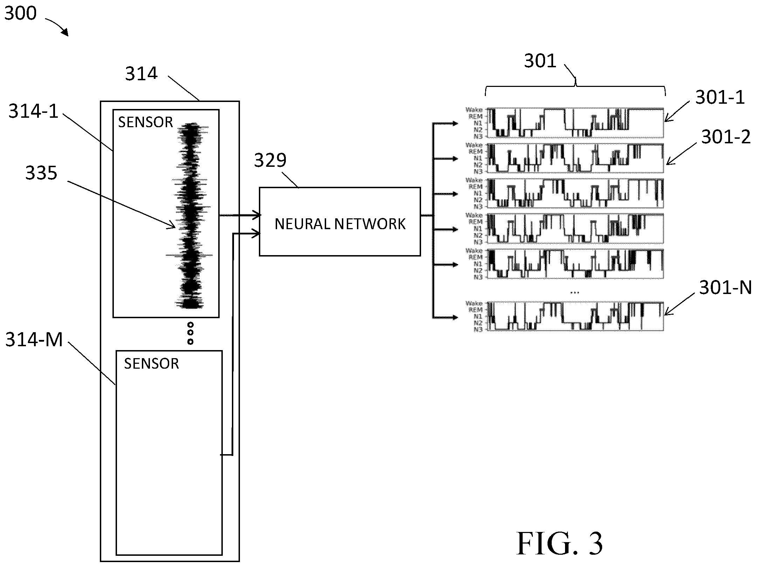

One or more controllers of the system 100 may employ machine learning and/or artificial intelligence (AI) engines to process information generated by the system, such as the SMI, as well as data provided to the system such as historical information. Further, the one or more controllers of the system 100 may be operative to detect for example, sleep stage probabilities (e.g., see, FIGs. 3-5), the presence or absence of sleep apnea, the severity of sleep apnea, etc., and/or may store and/or update results in a memory of the system (e.g., within the SMI), and may forward these results to the corresponding patient or clinician for their convenience as may be set forth in the SSI. The SMI may be accessed by the CSC 106, the MS 104, and/or the SMU 102 of the system for further review, processing, and/or analysis.

The SMU 102 may include one or more of a controller 120, a Tx/Rx 128, a memory 126, one or more sensors 114-1 through 114-J (generally sensor 114, where J is an integer), a neural network 129, and a user interface (UI) 118 with which a user, such as the patient or clinician, may interact. The controller 120 may control the overall operation of the SMU 102 and may communicate with the CSC 106, the MS 104, and/or the network 108 via the Tx/Rx 128. The controller 120 may include one or more logic devices such as a microprocessor ($\mu$P) 130 and may control the overall operation of the SMU 102. It should be appreciated that in some embodiments, the controller 120 may include digital and/or analog control circuitry. It is further envisioned that the SMU 102 may be a stand-alone unit with its own power supply.

The UI 118 may include a display 122 (e.g., a touch-screen display) and a user input interface 124 which may be combined with or separate from each other. The user input interface 124 may include a keyboard, a mouse, a trackball, touch sensitive sensor, and/or other device, such as a touchsensitive display, which may be stand alone or part of a system, such as part of a laptop, a personal digital assistant (PDA), a mobile phone (e.g., a smart phone), a smart watch, an e-reader, a monitor, a smart or dumb terminal or other device for communicating with the controller 120 via any operable link such as an wired and/or wireless communication link. The user input interface 124 may be operable for interacting with the controller 120 including enabling interaction within a UI 118 as described herein. Clearly the controller 120, the sensor 114, the user input interface 124, the user interface (UI) 118, the memory 126, and the network 108 may all or partly be a portion of a computer system or other device. The UI 118 may be operative to provide audio/visual feedback as well as graphical user interfaces (GUIs) to the user of the present system and may inform the operator of operating parameters, operating states, etc.

The controller 120 may include a microprocessor ($\mu$P) 130 and may be operative to render content, such as still or video information, as well as audio information on a rendering device of the system such as on the display 122 and/or speaker of the UI 118. This information may include information related to operating parameters, instructions, feedback, and/or other information related to an operation of the system, or portions thereof, such as SSI or portions thereof SMI, clinical decision support, application data, data generated by the system, operating parameters, etc., and/or combinations thereof. Clinical decision support (CDS) may be employed to analyze data generated by the system and may generate determinations which may be stored in the system and/or rendered for the convenience of the patient and/or clinician depending upon settings in the SSI. Where SSI may be system setting information that may be used by the system so set operational parameters and settings as may be set by the system and/or user.

The controller 120 may be operable for providing control signals and/or performing operations in response to input signals from the user input device 124 as well as in response to other devices of a network, such as the sensor 114, and executing instructions stored in the memory 126. The $\mu$P 130 may include one or more of a microprocessor, an application-specific and/or general-use integrated circuit(s), a logic device, etc. Further, the $\mu$P 130 may be a dedicated processor for performing in accordance with the present system and/or may be a general-purpose processor wherein only one of many functions operates for performing in accordance with the present system. The $\mu$P 130 may operate utilizing a program portion, multiple program segments, and/or may be a hardware device utilizing a dedicated and/or multi-purpose integrated circuit.

It is envisioned that one or more portions of the SMU 102, such as the controller 120, may be operationally coupled to the memory 126, the user interface (UI) 118 including a rendering device such as a display 122, the sensor 114, the user input interface 124, the neural network 124, and/or the network 108. The memory 126 may include any suitable memory configured to store information used or generated by the SMU 102, such as operating instructions, the SSI, the SMI, etc. In some embodiments, the memory 126 may store information related to test types, such as sensors employed by the test type, instructions for running the test type, the operating flow of the test type, the SSI and/or the SMI generated by the sensor 114, the test, test type, etc.

The neural network 129 may include any suitable neural network such as a stochastic neural network and/or other artificial neural network, etc. For example, the stochastic neural network is a type of artificial neural network built by introducing variations into the network. In accordance with the present system, the network is stochastic in the sense that multiple hypnograms may be derived from the same sleep information. This can be seen as the following function:

$$\text{hypnogram} = F(\text{measurement}, z), \qquad\qquad \text{Equation 1}$$

where z is a random variable that impacts the function of the neural network F in such a way that for each random draw of z, a different, but valid, hypnogram may be generated. Examples of such neural networks may include: generative adversarial network (GANs), Variational Autoencoders (VAEs), diffusion models, Monte-Carlo dropout, Bayesian neural networks, Normalizing Flows, etc. The neural network 129, may include a multi-plane neural network. The neural network 129 may include any suitable neural network and may be trained using stochastic machine learning models.

It is envisioned that embodiments of the present system may provide an automated method, based on stochastic machine learning models, for example, that provide uncertainty analysis of sleep parameters such as overnight sleep parameters. For example, the automated method in accordance with the present system may be achieved by having one or more models mimic the scoring biases of several different human scorers seen in the training set, thereby allowing the one or more models to output several different, but valid, hypnograms given the same input data. Similar as with multiple scorings made by humans, robust estimations (e.g., with mean or median) of sleep parameters may be calculated with these automatic systems. Additionally, measures of uncertainty may also be obtained, such as variance or correlation metrics. Accordingly, the neural network 129 may be trained on these models. The neural network as such may be described by means of a set of mathematical operations where, at each node of the network, inputs (from other nodes) are processed together with some predefined parameters (e.g., 'weights') to produce an output for that node. These weights are defined only once, during training, so they are not "inputs" per se, although of course need to be obtained (at least once) to build the desired neural network. This is traditionally done when developing and designing the software of the device and/or the system and comprises choosing a type of neural network architecture and choosing a so-called representative "training" set, based on which the weights are calculated. Once the network is trained, it may be deployed as part of the software running to process the sensor inputs (e.g., sleep information) in accordance with embodiments of the present system.

The neural network 129 may input sleep study signals via one or more channels and may calculate a variance for each sleep parameter. This variance may be rendered so that a clinical expert would be able to use the variance as input when making decisions. For example, the variance may be used to advise on continuing an ambulatory PSG and/or home sleep apnea test (HSAT) for an additional night when the measurement was not good enough (e.g., too much variance, such as variance beyond a predetermined acceptable amount), preventing additional logistic costs of returning the equipment when a desirability of further testing is indicated. Additionally, the variance of the sleep parameters may also be taken into account when calculating long-term trends. Moreover, the variance may also be used to mask or ignore certain measurements, when their removal leads to better performance of the automated model.

During operation, the neural network 129 may be configured to receive one sleep recording comprising one or multiple channels such as in a PSG, or HSAT, or alternatively, from surrogate sensors such as wearable PPG/actigraphy and/or nearable sensors, such as bed sensors, etc., and output several different but valid hypnograms. In accordance with embodiments of the present system, this may be achieved through the employment of one or a combination of different methods described in the literature such as Monte-Carlo Dropout as discussed in "Deepsleepnetlite: A Simplified Automatic Sleep Stage Scoring Model With Uncertainty Estimates" by L. Fiorillo et al., IEEE Transactions on Neural Systems and Rehabilitation Engineering, vol. 29, pp. 2076-2085, 2021. doi: 10.1109/TNSRE.2021.3117970; Bayesian Neural Networks as discussed in "Weight Uncertainty In Neural Network," by C. Blundell et al., in International Conference On Machine Learning. PMLR, 2015, pp. 1613-1622; Conditional Generative Models (e.g., Conditional GAN as discussed in "Conditional Generative Adversarial Nets" by M. Mirza and S. Osindero, arXiv preprint, 2014. doi: 10.48550/arXiv.1411.1784, Conditional Normalizing Flows as discussed in "Learning Likelihoods With Conditional Normalizing Flows" by C. Winkler et al., arXiv preprint, 2019. doi: 10.48550/arXiv.1912.00042, and Conditional Diffusion Models as discussed in "Conditional Image Generation With Score-Based Diffusion Models" by B. Georgios, et al., arXiv preprint, 2021); and Probabilistic U-Net "A Probabilistic U-Net For Segmentation Of Ambiguous Images" by S. Kohl et al., Advances In Neural Information Processing Systems, vol. 31, 2018, each of which is incorporated herein as if set out in entirety and operating in accordance with embodiments of the present system.

The memory 126 may be any type of device for storing application data as well as other data related to the described operation such as application data, data generated by the system, operating parameters, SSI, SMI, etc., one or more models related to the neural network 129, and/or combinations thereof. The application data, data generated by the system, operating parameters, SSI, SMI, etc., one or more models related to the neural network 129, and/or combinations thereof, may be received by the controller 120 for configuring (e.g., programming) the controller 120 to perform operation acts in accordance with the present system. The controller 120 so configured becomes a special purpose machine particularly suited for performing in accordance with embodiments of the present system.

The methods of the present system are particularly suited to be carried out by a computer software program, such program containing modules corresponding to one or more of the individual steps or acts described and/or envisioned by the present system. Such program may of course be embodied in a computer-readable medium, such as an integrated chip, a peripheral device or memory, such as the memory 126, and/or other memory coupled to the controller 120.

The program and/or program portions contained in the memory 126 may configure the controller 120 to implement the methods, operational acts, and functions disclosed herein. The memories may be distributed, for example between SMU 102, the MS 104 and/or the CSC 106, or local, and the controller 120, where additional processors may be provided, may also be distributed or may be singular. The memories may be implemented as electrical, magnetic, and/or optical memory, or any combination of these or other types of storage devices. Moreover, the term "memory" should be construed broadly enough to encompass any information able to be read from or written to an address in an addressable space accessible by the μP 130 of the controller 120. With this definition, information accessible through a network, such as the network 108, is still within the memory, for instance, because the μP 130 may retrieve the information from the network 108 for operation in accordance with embodiments of the present system.

The SMU 102 may include any suitable measurement system or systems (e.g., one or more sensors 114) to monitor the patient's physiological changes over time (e.g., epochs) during a (sleep) study period using one or more test types to determine physiological changes of the patient from which sleep features of the patient may be derived. These test types may have process flows and operations that may be stored in the SSI and may be selected by the SSI (e.g., automatically upon initiation), the patient, and/or the clinician as may be desired. In accordance with embodiments of the present system, it is envisioned that the SMU 102 may monitor the patient's physiology over a study period in accordance with one or more selected test types (e.g., each corresponding to a different type of sleep study) using one or more sensors 114, form corresponding sensor information which may be processed in accordance with the corresponding test type, and thereafter be stored as test results in the SMI. The study period may be a time period during which the patient is monitored. The test types may be changed and/or adjusted during the sleep study under the control of the controller 120.

Embodiments of the present system may be compatible with one or more test types, such as a Polysomnography (PSG) -type, a home sleep apnea test (HSAT), etc. Each of the test types may employ one of more sensors and/or sensor types with specific placement and operation that may correspond with the sensor and/or sensor test type. For example, while both Electroencephalography (EEG) and electromyography (EMG) test types may employ electrode type sensors (e.g., electrode patches, electrode bands, etc.), the EEG test they may be placed along the scalp of the patient to record bio signals representing electrical activity of the brain; and the EMG test may place the electrodes along, or on, the chest of the patient to record bio signals from skeletal muscles. These bio signals may then be processed in accordance with corresponding test type (e.g., the EMG) and stored in the SMI for later use.

Suitable test types (e.g., types of sleep studies) may include a home-type sleep study (e.g., the HSAT) using a simple test with few sensors such as used by the EMG type test that may monitor skeletal muscles, etc.), a Polysomnography (PSG) -type study which may monitor one or more bodily functions (e.g., for physiological changes) of the patient, such as brain activity (e.g., using an Electroencephalography (EEG) -type study), eye movements (e.g., using an Electrooculography (EOG) type study), muscle activity (e.g., using an electromyography (EMG) -type study), and heart rhythm (e.g., electrocardiography (ECG) -type study). From a given one or more tests/test types, corresponding sensor information may be formed, that subsequently, may be formed into an electrogram, electrooculogram, electromyogram, electrocardiogram, respectively, depending upon study type, and may be stored in the SMI for further processing. The process may apply machine learning algorithms or the like to analyze one or more of the electrogram, electrooculogram, electromyogram, electrocardiogram, and derive, or otherwise determine, a hypnogram which may include information for each sleep stage on a 30 second epoch over the testing period (e.g., sleep study).

One or more of the sensors 114 may be wirelessly coupled to the controller 120, if desired. It is also envisioned that the one or more sensors 114 may autonomously pair to the controller 120 upon determining that it is attached to a user and ready for use. One or more of the sensors 114 may be paired to the controller 120 by a clinician and/or the patient. It is also envisioned that one or more of the sensors 114 may be wearable and may be part of a PPG sensor suit of a wearable PPG-type device.

It is envisioned that, for example, when employing the EEG test type, one or more electrodes, such as may be provided by a Philips™ SmartSleep™ headband or the like, may be employed. In some embodiments, one or more functions of the patient during sleep may be tracked with other modalities either alone, and/or in combination with, for example the EEG, such as heart rate variability (e.g., measured with ECG, PPG, bed sensors, remote PPG cameras, etc.) or respiratory variability (e.g., measured with surrogate measures of respiratory effort, such as based on bioimpedance, thoracic and/or abdominal belts, PPG blood volume amplitude variations, bed sensors, radio-frequency (RF)/Doppler radar, infrared cameras, etc.). Thus, the one or more sensors of the system may vary based upon the test type and/or system and/or patient preferences. For example, one or more of the sensors 114 may vary in accordance with the (sleep) test type employed to track the one or more bodily functions of the patient during sleep. Similarly, the controller 120 may operate in accordance with the type of sleep test or tests employed to track the one or more signals corresponding to the physiological changes of the patient (e.g., as captured by the one or more sensors) over the study period and may process these signals in accordance with the test type.

In some embodiments, when for example performing a PSG-type test, the sensors may record one or more of eye movement, oxygen and/or carbon dioxide levels in the blood, heart rate, rhythm, breathing rate and rhythm, and airflow through the mouth and nose, snoring, etc., and form corresponding sensor information (e.g., by the sensor itself and/or in cooperation with the controller) that may be processed in accordance with the PSG-type test to determine the respiratory status of the patient which may then be stored in the SMI along with the processed signals, etc.

The sensor 114 may be situated at one or more portions of the system and may sense related parameters, form corresponding sensor information, and provide this sensor information to the controller 120 for further processing. For example, the sensor 114 may include one or more sensors which may be specific to a (sleep) test type being performed. When more than one sensor is being used, the sensor 114 may be distributed throughout the system 100.

The controller 120 may be configured to communicate with the MS 104 using any suitable communication method or methods such as via a Bluetooth™ connection or the like. The controller 120 may obtain the SSI from the MS 104 and/or the CSC 106 and may store this information in the memory 126 for reference at a later time and/or to configure its operation in accordance with the SSI. During operation, the controller 120 may, for example, receive analog signals from the sensors 114, amplify (e.g., using one or more amplifiers) and/or condition these analog signals (e.g., using one or more signal conditioners), convert these signals (e.g., using one or more analog-to-digital (A/D) converters) to digital signals (e.g., at a desired sampling rate (as may be set in the SSI)) to form corresponding information which may be further processed and/or may be stored in the SMI for later use and/or processing, such as by the neural network 129 to process them and output several different but valid hypnograms. In accordance with embodiments, the sensors may capture signals as digital signals and thereafter be suitably processed, stored, etc. For example, in accordance with embodiments, one or more of the sensors 114 may be an optical sensor (e.g., of a camera) that captures images of the patient as digital images that are processed, stored, etc. These hypnograms may then be further processed to, for example, determine the respiratory status of the patient.

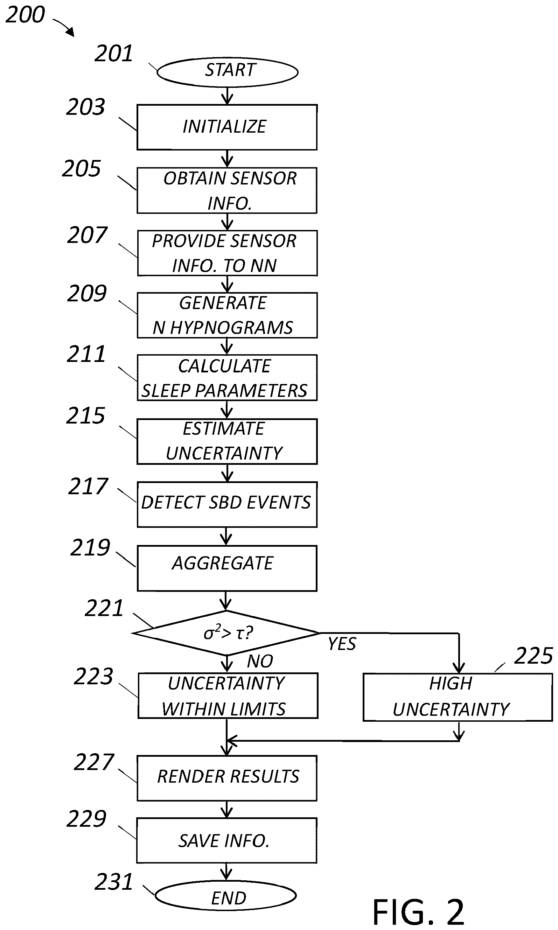

Operational processes performed by the system are now described with reference to FIG. 2 which illustratively shows a functional flow diagram performed by a process 200 in accordance with embodiments of the present system. The process 200 may be performed using one or more processors, computers, controllers, etc. communicating over a network and may obtain information from, and/or store information to, one or more memories which may be local and/or remote from each other. The process 200 may include one or more of the following acts. In accordance with embodiments of the present system, the acts of process 200 may be performed using a controller operating in accordance with embodiments of the present system. Further, one or more of these acts may be combined and/or separated into sub-acts, or performed serially or in parallel, as may be desired. Further, one or more of these acts may be skipped depending upon settings. For the sake of clarity, the process may be described with reference to a single SMU. However, without limitation, it should be understood that the process may employ a plurality of SMUs each of which may include a separate workflow such as a sub-workflow. In operation, the process 200 may start during act 201 and then proceed to act 203.

During act 203, the controller may be initialized by performing an initialization routine. During initialization, the process may load SSI and may set system settings in accordance with the SSI. The process may further obtain neural network models and/or algorithms that may process data generated by the process 200 in accordance with embodiments of the present system. The SSI may further include information related to the duration of the current sleep study period (e.g., eight hours, although other periods are also envisioned), epoch intervals (e.g., 30 seconds, although other epochs such as one minute, etc., are also envisioned), test process flow, test sensor parameters, and/or other settings. During this act, a test type may be set as may operating modes in accordance with settings in the SSI and/or from the clinician and/or the patient.

After completing act 203, the process (e.g., though operation of the controller 120) may continue to act 205 during which the controller may obtain sensor information corresponding to the sleep study. The sensor information may be obtained in real time from one or more of the sensors (e.g., sensor 114) or from a memory of the system. For example, the sensor information may be obtained in real time during the sleep study of the patient and/or may be obtained from a memory of the system (e.g., from the SMI), depending upon system settings as may be selected by the SSI, the clinician and/or the patient. Accordingly, if the sensor information is to be obtained in real time, the controller may monitor the sensors in accordance with the test type and obtain sensor information therefrom. In some embodiments, the controller may obtain sensor information indicative of the patient's physiological changes over time during the study period, using one or more test types, form corresponding sensor information which may be processed in accordance with the corresponding test type, and which thereafter may be stored as test results in the SMI. These test types may employ one of more sensor types (e.g., sensor modalities) with specific placement and/or operation that may correspond with the test type being performed. For example, when using EMG tests, the controller may obtain the sensor information from the EMG sensors such as surface EMG signals. Likewise, the controller may obtain an audio signal from a microphone and store this as audio information.

When using an EEG-type test, it is envisioned that the process may monitor the sleep of the patient using sensors with single or multiple electrodes. In some embodiments, it is envisioned that the Philips™ SmartSleep™ headband, smart watch, and/or smart ring (e.g., token), etc. may be employed. However, it is also envisioned that more extensive testing methods of different test types may be employed for more in-depth monitoring like PSG including EEG, EOG, and EMG. Alternatively, other test types may include further sleep features that may be tracked with other modalities either or in combination with EEG, such as heart rate variability (e.g., measured with ECG, PPG, bed sensors, remote PPG cameras, etc.) or respiratory variability (e.g., measured with surrogate measures of respiratory effort based on bioimpedance, thoracic and/or abdominal belts, PPG blood volume amplitude variations, bed sensors, RF/Doppler radar, infrared cameras, etc.). During the duration of the testing period, the process may monitor the sensors that are being employed, form corresponding sensor information, and store this information in a memory of the system, such as in the SMI, for further processing and analysis.

After completing act 205, the process may continue to act 207 during which the process may provide the sensor information to a neural network for processing. After completing act 207 the process may continue to act 209, during which the neural network may generate N output hypnograms from the sensor information, where N is an integer and represents the number of members (e.g., hypnograms) in the sample. N may be much larger than the number of input sensors. Since a form of posterior sampling may be performed: P(hypnogram| measurement), a sufficiently large number of N is selected for a good uncertainty estimate. A good value for N may be scientifically verified. However, when selecting a larger N, the computational expense (e.g., availability of computational resources and/or how long it takes to perform computations) is taken into account. For example, N may be determined when developing the software that will be included in the device/system in accordance with embodiments of the present system, and can effectively be any number between 3 (for some second-order statistics to be meaningful) and any larger number (e.g., bound only by availability of computational resources, and operating time).

Each of the hypnograms may include information related to sleep stages such as Wake, rapid eye movement (REM), N1, N2, and N3 sleep stages, although other stages (e.g., N4) are also envisioned. In accordance with previous (older) Rechtschaffen & Kales (R&K), there are six different stages of sleep namely: awake (W), non-rapid eye movement (N-REM) stage 1, N-REM stage 2, N-REM stage 3, N-REM stage 4, and the rapid eye movement (REM). However, in the current American Academy of Sleep Medicine (AASM) standard, there are only five (5) stages including wake: (W), REM, and (non-REM) N1-N3. This is illustrated with reference to FIG. 3 which illustratively shows a schematic block diagram of a portion of a system 300 operating in accordance with embodiments of the present system. One or more sensors 314-1 through 314-M (generally 314, where M is an integer) may generate sensor information, which may form all or part of a sleep recording 335. The sleep recording 335 may be a single input PSG or HSAT, or alternatively, may be obtained from surrogate sensors such as a wearable PPG/actigraphy or nearable sensors such as bed sensors or the like. The sensor information may then be provided to the neural network 329 for processing. The neural network is previously trained with training data (e.g., SMI selected from a population, at least some of which exhibit a studied physiological condition, e.g., OSA) and as discussed, is trained to produce a plurality of hypnograms, each of which is considered valid (e.g., similar as may be produced by a plurality of testing agents that analyzed the training data). For example, the neural network 329 may then generate N output hypnograms 301 such as hypnograms 301-1 through 301-N (generally 301) illustrated in graph 301. Each of these hypnograms 301 may be different from each other but may be valid.

Referring back to act 209 of FIG. 2, with regard to the N hypnograms, the system may select one or more groups or subgroups of hypnograms of the N hypnograms to determine the statistics (e.g., sleep statistics). Then statistics for each of these groups may be compared to each other and best performing group(s) may be selected. When comparing a group to a subgroup, the subgroup may be considered as the group with certain hypnograms excluded. This may be useful when trying to exclude erroneous data (e.g., data which may increase estimated uncertainty) that may be included in one or more of the hypnograms as discussed further herein. Illustratively, in the present embodiments it may be that all N hypnograms are selected. However, in other embodiments, a plurality of hypnograms may be selected randomly, due to resulting in less variance in the calculated sleep statistic(s), in accordance with one or more algorithms and/or selection methods as may be selected by the system, set in the SSI, selected by the clinician, and/or patient, etc. For example, this selection of hypnograms of the N hypnograms may enable hypnograms and their associated parameters to be selected (or excluded) for testing as discussed further herein.

After completing act 209, the process may continue to act 211 during which the process may calculate one or more sleep parameters in accordance with the hypnograms. Accordingly, after sampling selected ones of the N hypnograms, the process may calculate an estimate of one or more sleep statistics, for each corresponding hypnogram of the N hypnograms. For example, one or more of the sleep statistics may include: Sleep Onset Latency (SOL), Time to REM (TTR); Total Sleep Time (TST), Apnea-Hypopnea Index (AHI); time in each stage (in absolute terms and/or as percentage); Wake After Sleep Onset (WASO); Sleep Efficiency (SE); number of transitions between each stage; number of awakenings; number of awakenings from each stage; number of arousals; number of obstructive apneas; number of hypopneas; number of central apneas and/or index of any of the number of events indicated above, divided by the total sleep time, etc. It should be noted that this list is not exhaustive and could in principle be much longer and/or contain other factors. For example, in accordance with embodiments of the present system, any sleep parameter derived from the set of available input sensors may be utilized. This robust estimate may be obtained for example using sampling statistics. _For example, sample statistic such as mean, median, and may include an indication of deviation (variance or standard deviation, specific percentiles, interquartile ranges, etc.), etc. In addition, more sophisticated sampling methods such as bootstrapping, etc., may be used to generate statistics (such as those above) that are even more robust to the presence of outliers. In this way, "robustness" comes from the fact that multiple hypnograms are sampled, and from those, the desired sleep statistics (point estimates) are calculated. The sample statistics indicated above may be calculated to obtain robust estimates of those sleep statistics.

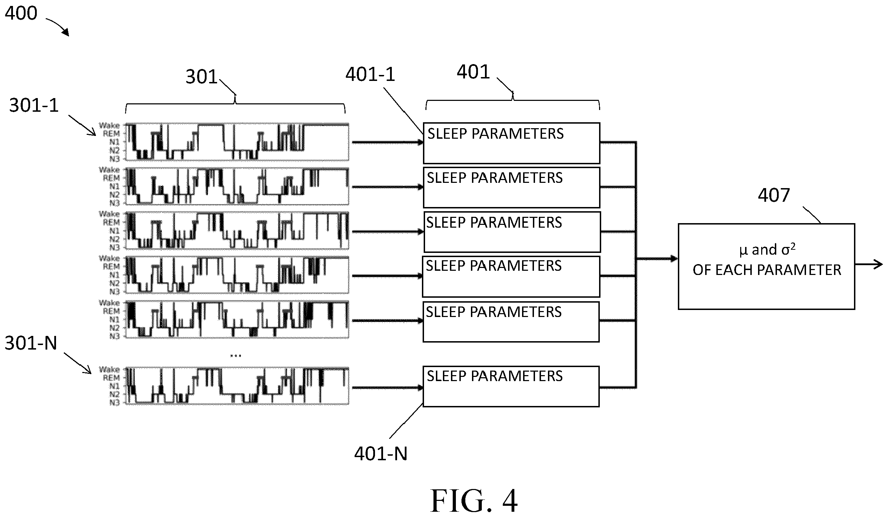

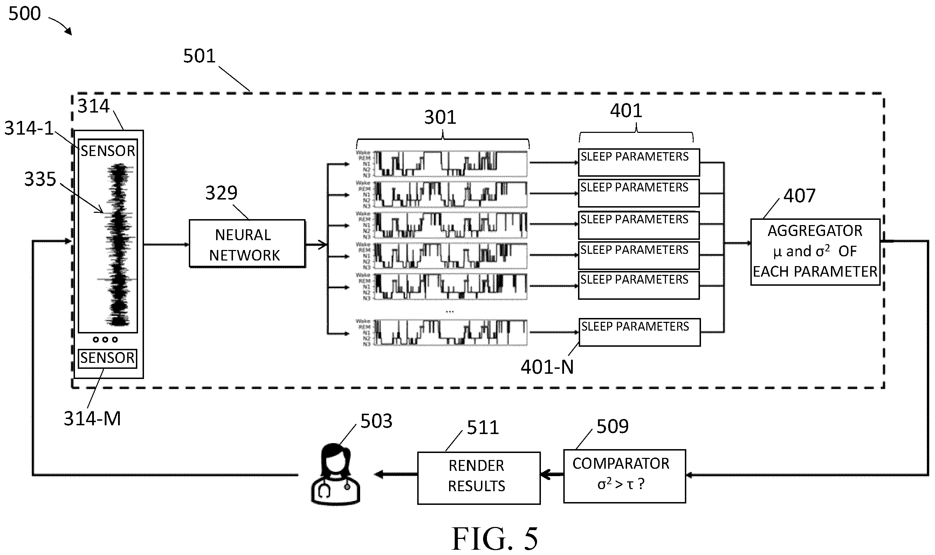

This is illustrated with reference to FIG. 4 which illustratively shows a schematic block diagram of a portion of a system 400 operating in accordance with embodiments of the present system. The N output hypnograms 301-1 through 301-N may be analyzed and sleep parameters 401-1 through 401-N, including (e.g., $\mu$ and $\sigma^2$) for each hypnogram, may be generated therefrom and represented as N point estimates 401 as sleep parameters. For any given plurality of histograms, only a point estimate (single scalar value) is calculated per statistic. These sleep parameters may then be forwarded to an aggregator 407 which determines a statistic(s) from the sleep parameters. For example, the aggregator 407 may determine $\mu$ which represents the mean of a given variable (e.g., of one or more aggregated/received sleep parameters) and the chi square statistic ($\sigma^2$) which represents the variance of the given variable in the sleep statistics (estimate of uncertainty Since each hypnogram provides a point estimate of each statistic(s), i.e., a single scalar value. The aggregator is configured to summarize all these results in terms of calculated statistics (e.g., aggregated statistics), such as mean, median, standard deviation, chi square statistic, etc. Additionally, kernel density estimation may be employed, and/or the statistics may be visualized for example using their empirical cumulative distribution functions. Thereafter, the aggregated statistics may be sent to a comparator such as is shown in FIG. 5.

FIG. 5 illustratively shows an automated sleep study system 500 for generating uncertainty estimates, such as may be used to advise whether or not further testing should be conducted, in accordance with embodiments of the present system. The system 500 may include an automated system 501 that may produce the uncertainty estimates, for example as illustrated with reference to the process shown in FIG. 2. As illustrated one or more sensors 314 (illustratively shown as sensors 314-1, ..., 314-M) may generate sensor information, which may form all or part of the sleep recording 335. The sleep recording 335 may be a single input PSG or HSAT, or alternatively, may be obtained from a plurality of sensors, such as a wearable PPG/actigraphy, sensors such as bed sensors or the like, sensors contained in smart watches, headbands, tokens, etc., that may collect sleep data during a sleep study/test to form the sleep recording 335. The sleep recording/data is received by the neural network 319 for processing to determine N output hypnograms 301.

In accordance with the present system, the neural network is previously trained with training data (e.g., SMI selected from a population, at least some of which exhibit a studied physiological condition, e.g., OSA) and as discussed, is trained to produce the plurality of hypnograms, each of which is considered valid. In accordance with embodiments of the present system, sleep parameters 401-1 through 401-N (generally, 401) may be calculated from the N output hypnograms 301, for example, including sleep parameters 401 for each hypnogram. The sleep parameters 401 may thereafter be forwarded to the aggregator 407 to be aggregated to calculate aggregated sleep parameters, as one or more sleep statistics, such as $\mu$ and the chi square statistic ($\sigma^2$) which represents the variance of the given variable in the sleep parameters (estimate of uncertainty). As shown, a comparator 509 may receive the calculated sleep statistic(s) forwarded from the aggregator 407 and compare one or more of the calculated sleep statistic(s) to a threshold (e.g., $\tau$). Based on the comparison, the results may be rendered (e.g., 511) and advice may be provided to the clinician 503 resulting in an adjustment to the system (e.g., sensors), further testing, etc., as discussed herein. In accordance with embodiments, the system 500 may be responsive to the advice (e.g., 509, 511) without the clinician 503 intervention, such as an adjustment of testing channels, further testing, etc., as discussed.

After completing act 211, the process may continue to act 215 during which the process may calculate an estimate of uncertainty ($\sigma^2$) associated with each of the calculated sleep statistics (e.g., see act 211). The estimate of uncertainty ($\sigma^2$) may vary due to many factors such as, faulty or noisy signals (e.g., poor electrode connection, and/or interference from electrical equipment, etc.), due to characteristics of the patient (dry or sweaty skin, etc.), measuring environment that may hamper the calculation of a given statistic such as pre-bedtime behavior (e.g. drugs/medication, alcohol, irregular bedtimes, etc.), specific pathology which may increase ambiguity related to e.g., sleep and wake (such as insomnia), poor connections to sensors, faulty sensors, radio-frequency (RF) interference (e.g., of wireless sensors, etc.), etc. This estimate may be obtained for example by using sample statistics such as variance, or in the case of multiple measurements, measurements of correlation or other statistics that may be calculated based upon the selected hypnograms, which illustratively and to simplify the discussion herein, is all of the N hypnograms However, in accordance with embodiments of the present system, it is envisioned that the statistics may be calculated for selected sample hypnograms as opposed to all N of the hypnograms. In accordance with embodiments of the present system, multiple samples of, for example, a hypnogram from the same set of inputs (e.g., from a single night). Further, in accordance with embodiments of the present system, multiple measurements sensor measurements from multiple nights may be utilized to produce multiple (hypnogram) samples from each night in the set of multiple night/measurements.

After completing act 215, the process may continue to act 217 during which the process may detect, for example, the presence of SDB events (e.g., for the selected hypnograms). For example, when using an HSAT (and/or other sleep study tests), the process may measure the presence and/or the severity of OSA, by means of estimating an AHI or any other comparable statistic, such as respiratory disturbance index (RDI), oxygen desaturation index (ODI), and/or respiratory event index (REI). Illustratively, AHI will be discussed for the sake of simplifying the discussion herein. However, it should be understood that other statistics such as RDI, ODI, and REI may be also calculated by the system as desired.

The AHI may be calculated using the total number of detected sleep-disordered breathing events (SDBs), divided by the total sleep time (TST). The SDBs are equal to the total of detected obstructive-sleep apneas (OSAs), central-sleep apneas (CSAs), hypopneas, etc., divided by the detected TST. Thus, the AHI may be represented as AHI = SDB/TST. Accordingly, the system may determine estimates for both measurements of SDBs (e.g., detection and count of SDB events in the sleep sample data) and the TST and may use these measurements to determine a number of estimates for the AHI summary statistic itself. For example, the system may produce N hypnograms with also N scorings of disordered breathing events. Thus yielding N estimates of the AHI, for example, which may be consequently aggregated into a $\mu$ and/or $\sigma^2$.

Thereafter, based upon the determined AHI summary statistics, the process may calculate a robust estimate of the statistic and an estimate of uncertainty $\sigma^2$. For the sake of clarity, embodiments are be described with respect to AHI. However, it should be understood that other measurements may be used, differing only on the type of events and the sleep parameters employed. In addition, besides determining the plurality of hypnograms, the present system may produce several samples of other sleep measurements including, e.g.: A- (temporal) location (i.e., when, during night they occur) and duration of arousals; B - location and duration of SDB events (including a possible differentiation between these, e.g., between obstructive apneas, hypopneas, central apneas); C- location, duration and degree of oxygen desaturations; and D - location, and duration of periodic limb movements. In accordance with embodiments, basically any sample that is traditionally produced based on sleep recordings may be utilized. These sleep measurements obviously describe different types of events, which can then lead to the computation of different sleep statistics. For example, from the examples above: - from A the present system may determine e.g., the number of arousals; - from B, e.g., the number of different types of SDB events (e.g., obstructive apneas, hypopneas, central apneas); - from C, the present system may derive the number of oxygen desaturations, possibly with different (traditional) desaturation thresholds (e.g., 3%, 4%); - from D, the present system may derive e.g., the number of periodic limb movements

Note that a combination of these measurements (and also, between these and samples of hypnogram) may be used to derive additional metrics; for example, in accordance with embodiments, the system may combine a sample estimate of the number of arousals (from A) with a sample estimate of total sleep time (from the hypnogram) to obtain a sample estimate of arousal index; idem for obstructive apnea index, hypopnea index, central apnea index; idem for oxygen desaturation index, or periodic limb movement index. In the same way, several of these could be combined together, possibly also with the hypnogram, to obtain even more complex sleep statistics. For example, the RDI (respiratory disturbance index) = AHI + #RERAs (respiratory effort related arousals)/total sleep time, where the number of RERAs may be obtained by monitoring respiratory effort, together with the indication (from A) of the location of arousals.

After completing act 217, the process may continue to act 219 during which the process may aggregate all the estimates of uncertainty and the means as illustrated by aggregator 407 in FIG. 4. After completing act 219, the process may continue to act 221 during which the process may compare the estimate of uncertainty $\sigma^2$ (determined during act 215) with one or more threshold values $\tau$ (e.g., see, FIG. 5, comparator 509). For example, the $\tau$ for a given threshold value may be determined from a validation set, where the present system plots the corresponding threshold against percentage of PSGs still included/excluded. Further, the present system may select a desired threshold (e.g., tau=0.5 means that 5% of recordings are rejected). Naturally one or more other thresholds and/or combinations of thresholds may also be suitably selected. Accordingly, in accordance with the present system, it does not necessarily have to be a single threshold that is compared. In accordance with embodiments, multiple parameters may be compared to multiple thresholds. For example, as in a stoplight system, a sigma<tau1 results in 'green light', tau1<sigma<tau2 results in 'orange light', and sigma>tau2 results in 'red light'. In addition, the present system may not only compare a determination to a threshold value in the decision support framework, but may also identify and determine the trend of parameters over time as discussed further herein. Additionally, the present system may take both mean and standard deviation into account with respect to some threshold. For example, there is an AHI cutoff at 15 events/hour. If the mean+- standard deviation allows for ambiguity whether or not that threshold is cleared, the present system may advise for additional testing.

For example, when it is determined that the estimate of uncertainty $\sigma^2$ is greater than the threshold value $\tau$, the process may continue to act 225. However, when it is determined that the estimate of uncertainty $\sigma^2$ is less than or equal to the threshold value r, the process may continue to act 227. The threshold value $\tau$ may be set by the system and/or clinician and may, for example, be stored in the SSI in a memory of the system.

During act 225, the process may perform actions for high estimates of uncertainty $\sigma^2$ such as to generate advisory information such as "advise to measure another night" so that the sleep study may be repeated to get better data that may have a lower estimate of uncertainty $\sigma^2$. The advisory sleep information may be rendered on a rendering device (e.g., see, act 227), such as a display, to advise the clinician/patient to continue testing for another night and/or the process may be automated wherein the sleep sensors (e.g., see, FIG. 1, sensor 114) are activated to continue testing in response to the advisory sleep information. After completing act 225, the process may continue to act 227 discussed further herein.

During act 223, the process may perform actions for estimates of uncertainty $\sigma^2$ that are within limits such as generate content indicating such. For example, the process may generate a report indicating that the "estimate of uncertainty $\sigma^2$ is within limits." After completing act 223, the process may continue to act 227 during which the process may render results of the uncertainty determinations such as "advise to measure another night," for high estimates of uncertainty $\sigma^2$; and the "estimate of uncertainty $\sigma^2$ is within limits," for low estimates of uncertainty, on a rendering device of the system such as on a display of the system. For example, the advisory sleep information may be rendered on the rendering device (e.g., see, act 225), such as the display, to advise the clinician/patient to continue/discontinue (e.g., see, act 223) testing for another night and/or the process may be automated wherein the sleep sensors (e.g., see, FIG. 1, sensor 114) are activated/deactivated to continue testing in response to the results of act 221. The process may further provide an application interface with which the patient or clinician may interact such as by selecting to view data generated by the present system as may be selected by the patient and/or clinician. For example, the clinician may view the hypnograms, the sleep parameters, the calculated sleep statistic(s), the estimates of uncertainty, SDBs, etc., as may be requested by the patient and/or clinician, (e.g., see, FIG. 5, 511).

Accordingly, results of the sleep apnea study may be rendered on a rendering device of the system. For example, a controller of the system may transmit information corresponding to the results of the process to a rendering device of the system (e.g., the display of the MS) which rendering device may then render this information and/or advisory information such as "high uncertainty, perform another study," or "recommendation for another night of measurement," "sensor X returned unreliable data, please reposition and continue testing another night" (e.g., when sensor data from a sleep study is unreliable), "measurements satisfactory, discontinue testing" or the like for the convenience of the viewer such as a clinician (e.g., see, FIG. 5, 503). The clinician may then determine whether to perform another sleep study and may inform the patient of such. The results of the sleep study may be transmitted to one or more devices of the system such as the CSC, the MS of the patient, and/or the MS of a clinician as may be set in the SSI and may be stored in the SMI (e.g., see, act 229) which also may be updated with the most recent test results. The process may further provide an interface such as a touch screen display with which the patient or clinician (e.g., see, FIG. 5, 503) may interact to, for example, view the results of the sleep study and data generated thereby.

After completing act 227, the process may continue to act 229 during which the process may save information generated by the system during the process such as the results of the sleep study, etc., in the SMI for later analysis and/or use. Accordingly, the SMI may be updated to reflect the results of the process (e.g., the current sleep study) and stored in a memory of the system. Accordingly, data generated by the process and/or results of the process may be stored in a memory of the system. After completing act 229, the process may continue to act 231 where it may end.

The recommendation for another night of measurement (e.g., one or more of acts 511 and/or produced by the clinician 503) is especially useful in ambulatory settings, where additional logistic costs of returning the equipment may be prevented if the measurements were not of sufficient quality. This may reduce costs and enhance patient convenience since testing may simply continue without a need to wait for a subsequent return of the testing equipment (e.g., see, FIG. 1, SMU 102). In this way, this estimate of uncertainty may be used by the system to provide the user with advice/direction on a course of action that depends on the application, with an aim of reducing the uncertainty of the summary statistics of interest.

Accordingly, embodiments of the present system may provide a sleep monitoring system, such as a home sleep monitoring system (e.g., a HSAT), which may determine to advise the clinician DME/prescriber/patient to instruct the patient to utilize the system, such as sensor(s), etc., for at least one additional night during which additional sleep data may be collected. DME refers to the supplier of the equipment, in healthcare systems (e.g. USA) where it is not the hospital/clinic making the (medical) system available to the patient and/or following up on usage. In other countries this entity is sometimes called home care provider (of medical equipment). After collection of the additional night(s) sleep data, the system may then recalculate one or more statistics of interest and associated estimate of uncertainty using, for example, the sleep data from the additional night, or may use at least some portion of the new sleep data in combination with at least some portion of the previously collected sleep data (e.g., sensor data that is noisy in the new and/or previously collected sleep data may be removed from consideration when calculating the one or more statistics of interest and associated estimate of uncertainty.

When the uncertainty is determined to have decreased in or together with the new data, then the monitoring may stop and the system may render an indication of such on a rendering device of the system. Conversely, when it is determined that the uncertainty has not decreased, the system may render such information to inform the clinician. In that event, the clinician may then decide to use the current system (e.g., HSAT) for one or more additional night(s), may decide to change one or more sensor(s) and/or sensor type(s), and/or may decide to change the monitoring system to something inherently less uncertain (e.g., with additional physiological channels, the physician may decide to finally bring the patient in for an in-lab PSG for a more accurate (less uncertain) statistic to guide the diagnosis. In this way, the present system may provide an important tool for the clinician to assess the patient and/or the performance of the present system.

In accordance with embodiments of the present system, the threshold ($\tau$) chosen may vary depending on the goal or context in which the system is in operation. For example, for a patient where it is especially important to get a correct apnea diagnosis, the estimate of uncertainty ($\sigma^2$) threshold for the AHI may be set to a lower value so as to provide a stricter threshold of uncertainty than for other less relevant statistics. In case wherein the estimate of uncertainty is higher than the threshold, the system may advise the referring physician to continue the measurement for an additional night, change one or sensors and/or sensor types, etc. In the event that other changes have not helped decrease the uncertainty, the system may advise the clinician to have the patient brought in for an in-lab PSG for a more accurate (less uncertain) statistic to guide the diagnosis.

In accordance with embodiments, in place of recommending to measure a whole additional night when the variance of one of the sleep parameters is too high, a search strategy may be provided by the system to drop (e.g., exclude) one or more input channels with too high noise (e.g., uncertainty in the measured physiological signal). For example, the system may drop one or more channels by greedily dropping (e.g., excluding) input channels into the system and determining whether or not the variance (e.g., uncertainty) decreases. In a case wherein it is determined that the variance has decreased (e.g., after a channel is dropped), the system may tag the dropped input channel to indicate that it should not be employed for further testing with the patient. However, when it is determined that the variance has not decreased, the system may reuse the channel and drop another channel for testing either randomly, in groups and/or in a certain order. In accordance with the present system, when the variance does not decrease by removing one or more input channels, then it may be determined that uncertainty really is inherent and another one or more nights of measurements may be advised. Accordingly, the system may render information of such and may be set to perform another sleep study on the patient. However, when it is determined that the variance does indeed decrease by removing an input channel, it can be interpreted that this channel is too noisy or contains too many artifacts to use for sleep data collection. Accordingly, the system may render information of such to inform the clinician or patient of this and/or may autonomously remove the channel that is determined to be too noisy from further testing and/or analysis to thereby lower the variance/uncertainty.

This can happen concretely when performing a sleep study, in particular, at home in a setting without supervision of an experienced clinician (as opposed to an in-lab PSG where clinicians may be present), where it often occurs that some input channels (e.g., of ambulatory PSG, HSATs, etc.) are unreliable (e.g., with high impedance due to poor contact with the body, or impacted by excessive body movements associated with, for example, pathologies like sleep movement disorders). In this case, the system may generate, as described above, a measure of uncertainty for combinations of input channels. Accordingly, embodiments of the present system may be configured to automatically determine the set of channels (e.g., sensor channels) that lead to the highest performance (or lowest uncertainty). In this way, embodiments of the present system may be used to: a) produce a more robust estimate of the statistic of interest; and b) advise the patient or clinician that a specific input channel is unreliable and will not be considered in the analysis of the signal. In accordance with the present system, this information may be useful for the patient and/or clinician (e.g., a referring physician) and may be transmitted to the clinician to be rendered on a display of the system such as an MS of the clinician.

In a similar vein to dropping channels when determined to contain too much noise or artifacts and thus decrease the performance of the model, modalities may also be selected and/or removed. For example, assuming that both PPG and respiratory effort are measured during a sleep study, but the subject is taking medication that impacts heartrate but not breathing. The system may determine this when so informed (e.g., within the SSI, by the clinician, by the patient, etc.) and drop the PPG measurement which should then result in a lower variance of the output of the automated model. In this embodiment the first night of monitoring may include multiple sensors that may either be part of the same recording system (e.g., ambulatory PSG, etc.) or may be separate physical sensors (e.g., a wearable PPG device, a bed sensor device under the mattress, and/or sensors integrated in a therapy device like the airflow sensor of a CPAP). It is envisioned that embodiments of the system may determine a suitable monitoring modality for prolonged monitoring at home (e.g., during follow-up to therapy) and may determine and render recommendations, based on the initial measurement(s), which sensors would likely be optimal for monitoring, such as beyond the first assessment.

When studying trends of sleep parameters over several nights the proposed system/method allows for taking the uncertainty of the statistics into account. In this way then, options may be presented such as: (1) entirely remove the measurements of those nights with too high a variance by isolating or removing the channels and/or sensors, automatically by the system and/or through user intervention, that are determined to have low confidence; or (2) to take the variance into account when calculating the hypnograms, calculating statistics, etc. In a setup wherein more than one channel is provided for one or more sensors, a channel that presents too high a variance may be removed or may be substituted with a channel that provides a lower variance. In this way, the present system may help prevent false detections based on sudden increasing trends (e.g., last few nights the patient had suddenly a very high AHI, but the estimate of uncertainty is extremely high). In these cases, the system/method of the present system may detect this and, in response may, depending upon system settings, inform a clinician, and provide information related to this detected uncertainty such as information indicating such to be rendered on a rendering device of the system and/or remove/substitute channels that provide too high a variance. Alternatively, it is envisioned that the system may be configured to obtain additional measurements (e.g., wait a few more nights, perform more nights of testing, etc.) before alerting the responsible user depending upon system configuration.

Embodiments of the present system may be integrated into various sleep apnea solutions and sleep monitoring solutions. Next to the home monitoring setting, the solution may also be used to screen for sleep apnea in hospitals and other care settings. As operations may be automated, a clinician may not be necessary for performing sleep analysis using embodiments of the present system. Further, embodiments of the present system may, depending upon embodiment, employ fewer sensors than employed by conventional systems which enhances user convenience and reduces costs over prior systems.

Further variations of the present system would readily occur to a person of ordinary skill in the art and are encompassed by the following claims.

Finally, the above discussion is intended to be merely illustrative of the present system and should not be construed as limiting the appended claims to any particular embodiment or group of embodiments. Thus, while the present system has been described with reference to exemplary embodiments, it should also be appreciated that numerous modifications and alternative embodiments may be devised by those having ordinary skill in the art including using features that are described with regard to a given embodiment with other envisioned embodiments without departing from the broader and intended spirit and scope of the present system as set forth in the claims that follow. In addition, any section headings included herein are intended to facilitate a review but are not intended to limit the scope of the present system. In addition, the specification and drawings are to be regarded in an illustrative manner and are not intended to limit the scope of the appended claims.

In interpreting the appended claims, it should be understood that:

a) the word "comprising" does not exclude the presence of other elements or acts than those listed in a given claim;

b) the word "a" or "an" preceding an element does not exclude the presence of a plurality of such elements;

c) any reference signs in the claims do not limit their scope;

d) several "means" may be represented by the same item or hardware or software implemented structure or function;

e) any of the disclosed elements may be comprised of hardware portions (e.g., including discrete and integrated electronic circuitry), software portions (e.g., computer programming), and any combination thereof;

f) hardware portions may be comprised of one or both of analog and digital portions;

g) any of the disclosed devices, features and/or portions thereof may be combined together or separated into further portions unless specifically stated otherwise;

h) no specific sequence of acts or steps is intended to be required unless specifically indicated; and

i) the term "plurality of" an element includes two or more of the claimed element, and does not imply any particular range of number of elements; that is, a plurality of elements may be as few as two elements, and may include an immeasurable number of elements.

## Claims

1. A sleep monitoring system (100, 500), comprising:

at least one sensor (114) configured to sense physiological changes of a subject during a sleep study period having a plurality of epochs and form corresponding sleep study information for the plurality of epochs during the sleep study;

a stochastic neural network (129, 329) configured to:

receive the sleep study information for the plurality of the epochs during the sleep study period from the at least one sensor; and
derive a plurality of sleep sample evaluations each different from each other and based on a same sampling of the received sleep study information;

at least one controller (120, 190, 194) configured to:

receive the plurality of sleep sample evaluations and calculate a robust estimate of one or more sleep parameters thereon;
calculate an estimate of uncertainty for at least one of the one or more sleep parameters;
determine whether the estimate of uncertainty for the at least one of the one or more sleep parameters is greater than a threshold value; and
render the estimate of uncertainty on a rendering device (192) of the system based upon the determination.

2. The sleep monitoring system of claim 1, wherein the plurality of sleep sample evaluations are a plurality of hypnograms, wherein the at least one controller is further configured to analyze the plurality of hypnograms to determine a total number of obstructive and central apneas and hypopneas, and total sleep time (TST) for each of the plurality of hypnograms.

3. The sleep monitoring system of claim 2, wherein the at least one controller is further configured to receive the plurality of sleep sample evaluations and calculate a robust estimate of two or more sleep parameters thereon and calculate an estimate of uncertainty for at least two of the two or more sleep parameters.

4. The sleep monitoring system of claim 3, wherein the at least one controller is further configured to receive the plurality of sleep sample evaluations covering at least two sleep study nights and calculate a robust estimate of one or more sleep parameters thereon, wherein the at least one controller is further configured to identify a trend of the one or more sleep parameters over time.

5. The sleep monitoring system of claim 1, wherein the at least one controller is further configured to repeat the sleep study when the estimate of uncertainty associated with one or more of the one or more sleep parameters is greater than a threshold value.

6. The sleep monitoring system of claim 1, wherein the plurality of sleep sample evaluations are at least one of: A - (temporal) location (i.e., when, during night they occur) and duration of arousals; B - location and duration of SDB events (including a possible differentiation between these, e.g., between obstructive apneas, hypopneas, central apneas); C- location, duration and degree of oxygen desaturations; D - location, and duration of periodic limb movements; E - a plurality of hypnograms, wherein the at least one controller is further configured to calculate the robust estimate of one or more sleep parameters and to calculate the estimate of uncertainty for each of the plurality of sleep parameters.

7. The sleep monitoring system of claim 6, wherein the at least one controller is further configured to calculate one or more combinations of the sleep sample evaluations and to calculate a robust estimate of one or more combinations of the sleep sample evaluations to calculate an estimate of uncertainty for each of the plurality of combinations of the sleep sample evaluations.

8. The sleep monitoring system of claim 7, wherein the at least one controller is further configured to determine whether the aggregated estimate of uncertainty for each of the one or more sleep parameters is greater than a corresponding threshold value.

9. The sleep monitoring system of claim 8, wherein the at least one controller is further configured to render the estimate of uncertainty on the rendering device of the system based upon the determination of whether the aggregated estimate of uncertainty for one or more of the one or more sleep parameters is greater than the corresponding threshold value.

10. The sleep monitoring system of claim 9, wherein the at least one controller is further configured to repeat the sleep study when the aggregated estimate of uncertainty associated with the one or more of the one or more sleep parameters is greater than the corresponding threshold value.

11. The sleep monitoring system of claim 8, wherein the at least one controller is further configured to render the estimate of uncertainty on the rendering device of the system and a recommendation to add at least one additional sensor or substitute a second sensor for one of the at last one sensor based upon a determination that the aggregated estimate of uncertainty for one or more of the one or more sleep parameters is greater than a corresponding threshold value, wherein the at least one controller is further configured to repeat the sleep study with the added at least one additional sensor or the substituted second sensor when the aggregated estimate of uncertainty associated with the one or more of the one or more sleep parameters is greater than the corresponding threshold value.

12. A sleep monitoring system (100, 500), comprising:

at least one sensor (114) configured to sense physiological changes of a subject during a sleep study period having a plurality of epochs and form corresponding sleep study information for the plurality of epochs during the sleep study, wherein the sleep study information comprises a plurality of channels;
a stochastic neural network (129, 329) configured to:

receive the sleep study information for the plurality of the epochs during the sleep study period from the at least one sensor; and
derive a plurality of hypnograms each different from each other and conditioned upon the received sleep study information;

at least one controller (120, 192, 194) configured to:

receive the plurality of sleep sample evaluations and calculate a robust estimate of one or more sleep parameters thereon;
calculate an estimate of uncertainty for at least one of the one or more sleep parameters;
determine whether the estimate of uncertainty for the at least one of the one or more sleep parameters is greater than a threshold value;
render the determination when the estimate of uncertainty for the at least one of the one or more sleep parameters is equal to or less than the threshold value; and
delete one of the plurality of channels from the sleep study information for one or more of the plurality of the epochs to derive modified sleep study information when the estimate of uncertainty for one or more of the one or more sleep parameters is determined to be greater than the threshold value,

wherein the stochastic neural network is further configured to derive a second plurality of sleep sample evaluations each different from each other and conditioned upon received modified sleep study information, and wherein the at least one controller is further configured to:

receive the second plurality of sleep sample evaluations and calculate a robust modified estimate of one or more sleep parameters thereon,
calculate a modified estimate of uncertainty for at least one of the one or more modified sleep parameters,
determine whether the modified estimate of uncertainty for the at least one of the one or more modified sleep parameters is greater than the threshold value, and
render the determination when the modified estimate of uncertainty for the at least one of the one or more modified sleep parameters is equal to or less than the threshold value.

13. The sleep monitoring system of claim 12, wherein when the modified estimate of uncertainty for the at least one of the one or more modified sleep parameters is greater than the threshold value, the at least one controller is further configured to replace the one of the plurality of channels back into the sleep study information and delete a second one of the plurality of channels from the sleep study information, the stochastic neural network is further configured to derive a third plurality of sleep sample evaluations each different from each other and conditioned upon received further modified sleep study information, and the at least one controller is further configured to determine whether a further modified estimate of uncertainty for the at least one of the one or more further modified sleep parameters is greater than the threshold value.

14. The sleep monitoring system of claim 13, wherein the at least one controller and the stochastic neural network are further configured to replace and utilize different ones of the plurality of channels until each one of the plurality of channels have been replaced unless a resultant estimate of uncertainty for the at least one of the one or more sleep parameters is equal to or less than the threshold value.

**15.** The sleep monitoring system of claim 12, wherein the at least one controller is further configured to receive the plurality of sleep sample evaluations and calculate a robust estimate of two or more sleep parameters thereon and calculate an estimate of uncertainty for at least two of the two or more sleep parameters.

FIG. 1

EP 4 388 985 A1

*200*

*201* → ( START )

*203* → INITIALIZE

*205* → OBTAIN SENSOR INFO.

*207* → PROVIDE SENSOR INFO. TO NN

*209* → GENERATE N HYPNOGRAMS

*211* → CALCULATE SLEEP PARAMETERS

*215* → ESTIMATE UNCERTAINTY

*217* → DETECT SBD EVENTS

*219* → AGGREGATE

*221* → $\sigma^2 > \tau?$

YES

NO

*223* → UNCERTAINTY WITHIN LIMITS

*225* → HIGH UNCERTAINTY

*227* → RENDER RESULTS

*229* → SAVE INFO.

*231* → ( END )

FIG. 2

FIG. 3

FIG. 4

FIG. 5

Europäisches
Patentamt

European
Patent Office

Office européen
des brevets

## PARTIAL EUROPEAN SEARCH REPORT

**Application Number**

under Rule 62a and/or 63 of the European Patent Convention.
This report shall be considered, for the purposes of
subsequent proceedings, as the European search report

EP 23 15 3931

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/070935 A1 (NAT UNIV SINGAPORE [SG]) 19 April 2018 (2018-04-19) | 1,5-11 | INV.<br>A61B5/00<br>A61B5/024 |
| Y | * paragraph [0009] *<br>* paragraph [0020] *<br>* paragraph [0063] - paragraph [0069] *<br>* paragraph [0083] - paragraph [0087] *<br>* paragraph [0094] - paragraph [0098] *<br>* figures 2, 5A, 9 * | 2-4 | |
| Y | US 2020/405222 A1 (DE GROOT KOEN THEO JOHAN [NL] ET AL) 31 December 2020 (2020-12-31)<br>* paragraph [0035] - paragraph [0049] * | 2-4 | |
| X | US 2020/107775 A1 (DE CHAZAL PHILIP [AU] ET AL) 9 April 2020 (2020-04-09)<br>* paragraph [0066] - paragraph [0067] *<br>* paragraph [0087] - paragraph [0090] *<br>* figure 4 * | 1,2,6-11 | |

**TECHNICAL FIELDS SEARCHED (IPC)**

A61B

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 20 October 2023 | Gooding Arango, J |

EPO FORM 1503 03.82 (P04E07)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................
& : member of the same patent family, corresponding document

Europäisches
Patentamt

European
Patent Office

Office européen
des brevets

**INCOMPLETE SEARCH
SHEET C**

```
Claim(s) completely searchable:
      1-11

Claim(s) not searched:
      12-15

Reason for the limitation of the search:

According to the selection of the applicant in response to invitation
pursuant to Rule 62a(1) EPC, the search is based on claims 1-11.
```

## ANNEX TO THE EUROPEAN SEARCH REPORT
## ON EUROPEAN PATENT APPLICATION NO.

EP 23 15 3931

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2023

| Patent document cited in search report | | Publication date | Patent family member(s) | | Publication date |
|---|---|---|---|---|---|
| WO 2018070935 | A1 | 19-04-2018 | SG 10201608507P | A | 30-05-2018 |
| | | | WO 2018070935 | A1 | 19-04-2018 |
| US 2020405222 | A1 | 31-12-2020 | CN 111867450 | A | 30-10-2020 |
| | | | EP 3536225 | A1 | 11-09-2019 |
| | | | EP 3761852 | A1 | 13-01-2021 |
| | | | JP 2021517008 | A | 15-07-2021 |
| | | | US 2020405222 | A1 | 31-12-2020 |
| | | | WO 2019170734 | A1 | 12-09-2019 |
| US 2020107775 | A1 | 09-04-2020 | US 2020107775 | A1 | 09-04-2020 |
| | | | WO 2020072434 | A1 | 09-04-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **L. FIORILLO et al.** Deepsleepnetlite: A Simplified Automatic Sleep Stage Scoring Model With Uncertainty Estimates. *IEEE Transactions on Neural Systems and Rehabilitation Engineering,* 2021, vol. 29, 2076-2085
- **C. BLUNDELL et al.** Weight Uncertainty In Neural Network. *International Conference On Machine Learning. PMLR,* 2015, 1613-1622

- **M. MIRZA ; S. OSINDERO.** *Conditional Generative Adversarial Nets,* 2014
- **C. WINKLER et al.** *Learning Likelihoods With Conditional Normalizing Flows,* 2019
- **B. GEORGIOS et al.** *Conditional Image Generation With Score-Based Diffusion Models,* 2021
- **S. KOHL et al.** A Probabilistic U-Net For Segmentation Of Ambiguous Images. *Advances In Neural Information Processing Systems,* 2018, vol. 31